(19) Europäisches Patentamt / European Patent Office / Office européen des brevets

(11) **EP 3 739 376 B1**

(12) **EUROPEAN PATENT SPECIFICATION**

(45) Date of publication and mention
of the grant of the patent:
**29.04.2026  Bulletin 2026/18**

(51) International Patent Classification (IPC):
*G02C 13/00* (2006.01)    *A61B 3/00* (2006.01)
*G09G 5/00* (2006.01)    *A61B 3/113* (2006.01)

(21) Application number: **18899881.9**

(22) Date of filing: **11.01.2018**

(52) Cooperative Patent Classification (CPC):
**A61B 3/0025; A61B 3/0041; A61B 3/032;
A61B 3/04; A61B 3/113; G06F 3/147**

(86) International application number:
**PCT/JP2018/000528**

(87) International publication number:
**WO 2019/138515 (18.07.2019 Gazette 2019/29)**

(54) **IMAGE FORMING DEVICE, EYEGLASS LENS SELECTION SYSTEM, IMAGE FORMING METHOD, AND PROGRAM**

BILDERZEUGUNGSVORRICHTUNG, BRILLENGLASAUSWAHLSYSTEM, BILDERZEUGUNGSVERFAHREN UND PROGRAMM

DISPOSITIF DE FORMATION D'IMAGE, SYSTÈME DE SÉLECTION DE VERRE DE LUNETTES, PROCÉDÉ DE FORMATION D'IMAGE, ET PROGRAMME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**18.11.2020  Bulletin 2020/47**

(73) Proprietor: **Nikon-Essilor Co., Ltd.
Tokyo 130-0026 (JP)**

(72) Inventor: **CHO, Sungjin
Tokyo 130-0026 (JP)**

(74) Representative: **Hoffmann Eitle
Patent- und Rechtsanwälte PartmbB
Arabellastraße 30
81925 München (DE)**

(56) References cited:
WO-A1-2009/122684    WO-A1-2010/044383
WO-A1-2013/067230    WO-A1-2017/047178
JP-A- 2009 230 699    JP-A- 2010 134 460
JP-A- 2015 522 834    JP-A- H07 261 724
US-A1- 2016 270 656    US-A1- 2017 052 393
US-A1- 2017 052 393

## Description

### TECHNICAL FIELD

The present invention relates to an image generating device, an eyeglass lens selection system, a method for image generation and a program.

### BACKGROUND ART

Various methods that may be adopted when generating an image of the visual field to be viewed through a virtual eyeglass lens by an eyeglass lens wearer have been proposed. For instance, Japanese Patent No. 6023801 hereinafter PTL 1 discloses a device to be worn on the head of the wearer, which utilizes information obtained via a range image sensor. A device with a simpler structure than this, capable of providing the subject with an experience closely simulating the reality of looking at the visual field through an eyeglass lens, is eagerly awaited.

Each of WO 2013/067 230 and US 2016/270 656 A1 forms part of the state of the art relative to the present disclosure.

Document US-A-2017/052393 discloses an eyeglass lens comparison simulation system using virtual reality headset including: a first user terminal configured to receive at least one parameter that relates to one or more virtual eyeglass lens images; and a second user terminal configured to receive the at least one parameter from the first user terminal and produce virtual user-customized eyeglass lens images and virtual vision control effect images based on the at least one parameter received from the first user terminal, the virtual vision control effect images obtained by overlaying the virtual user-customized eyeglass lens images on real environmental images or virtual environmental images. The second user terminal can include a motion sensor which senses the motion of the second user terminal and outputs the sensed signal. The controller changes and outputs the virtual vision control effect images in response to the sensed signal received from the motion sensor.

### SUMMARY OF INVENTION

According to the 1st aspect of the present invention, there is provided an image capturing device as recited in claim 1 below.

According to the 2nd aspect of the present invention, there is provided an eyeglass lens selection system as recited in claim 11 below.

According to the 3rd aspect of the present invention, there is provided a method for image generation as recited in claim 12 below.

According to the 4th aspect of the present invention, there is provided a program as recited in claim 13 below.

### BRIEF DESCRIPTION OF DRAWINGS

[Fig. 1] Fig. 1 is a conceptual diagram schematically illustrating the structure of the image generating device according to an embodiment.
[Fig. 2] Fig. 2(A) provides a schematic illustration of an image obtained by capturing an image of the subject's face and Fig. 2(B) provides a conceptual illustration showing features of the subject's face.
[Fig. 3] Fig. 3(A) illustrates the range of the visual field viewed by the subject through an eyeglass lens and Fig. 3(B) provides a schematic illustration of an example of a display image in the case of Fig. 3(A).
[Fig. 4] Fig. 4(A) illustrates the range of the visual field viewed by the subject through an eyeglass lens and Fig. 4(B) provides a schematic illustration of an example of a display image in the case of Fig. 4(A).
[Fig. 5] Fig. 5(A) is a conceptual diagram indicating the positions of objects in a virtual space and Fig. 5(B) provides a schematic illustration of an example of a display image in the case of Fig. 5(A).
[Fig. 6] Fig. 6(A) is a conceptual diagram indicating the positions of objects in a virtual space and Fig. 6(B) provides a schematic illustration of an example of a display image in the case of Fig. 6(A).
[Fig. 7] Fig. 7 is a conceptual diagram illustrating a method for applying skew to an image of a virtual visual field.
[Fig. 8] Fig. 8 is a conceptual diagram illustrating a method for applying skew to an image of a virtual visual field.
[Fig. 9] Fig. 9(A) is a conceptual diagram presenting an example of a method for generating a blurred image without direction dependence and Fig. 9(B) is a conceptual diagram presenting an example of a method for generating a blurred image with direction dependence.
[Fig. 10] Fig. 10 is a conceptual diagram illustrating how the kernel may change based upon accommodation.
[Fig. 11] Fig. 11 presents a flowchart showing a flow of a method for manufacturing an eyeglass lens according to an embodiment that includes the method for image generation in the embodiment.
[Fig. 12] Fig. 12 is an illustration of a program according to an embodiment.

### DESCRIPTION OF EMBODIMENT

The following is a description of an image generating device and the like achieved in an embodiment, given in reference to the drawings as needed. In the following description, the terms "upper side", "lower side", "upper region", "lower region" and the like used with respect to an eyeglass lens denote areas within the lens viewed from a wearer of the eyeglass lens.

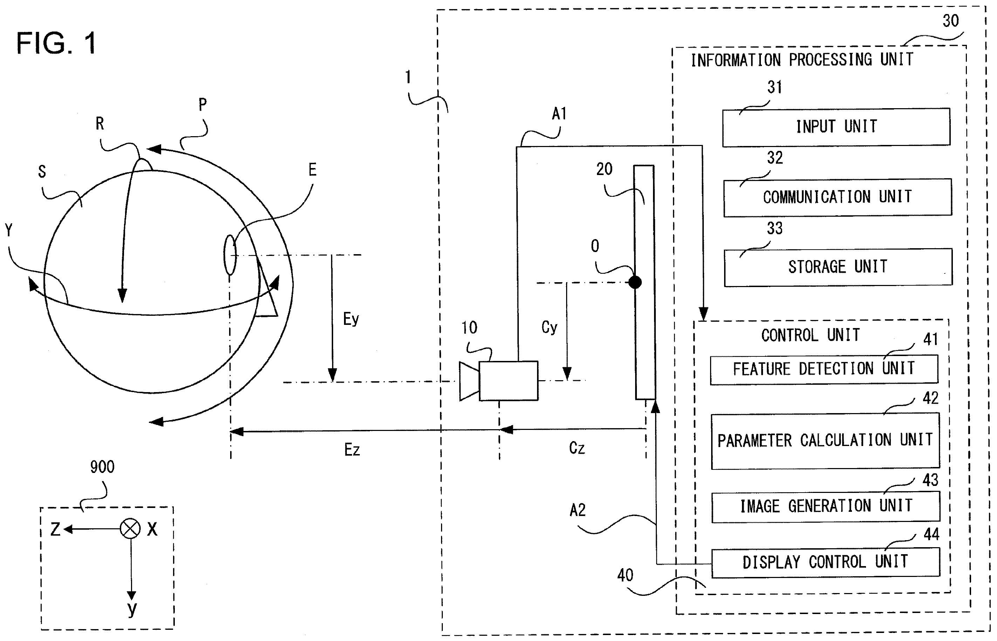

Fig. 1 is a conceptual diagram schematically illustrating the structure of the image generating device achieved in the embodiment. An image generating device 1 comprises an image capturing unit 10, a display unit 20 and an information processing unit 30. The information processing unit 30 comprises an input unit 31, a communication unit 32, a storage unit 33 and a control unit 40. The control unit 40 comprises a feature detection unit 41, a parameter calculation unit 42, an image generation unit 43 and a display control unit 44.

The image generating device 1 generates an image of a virtual visual field (hereafter will be referred to as a visual field image), viewed by a subject S looking at the display unit 20 through a virtual eyeglass lens, based upon eyeglass lens shape data and the like.

The image capturing unit 10, which comprises an image capturing device such as a camera, captures an image of the head of the subject S and, more specifically, the face of the subject S. As long as features needed for the processing executed by the parameter calculation unit 42, as will be explained later, can be detected in an image obtained through image capturing, the image capturing unit 10 may be disposed at any position. Light (image) having entered the image capturing unit 10 is converted to signals indicating light intensity through photoelectric conversion, then the signals undergo A/D conversion and the resulting signals are input to the control unit 40 (arrow A1).

The display unit 20, which comprises a display device such as a liquid crystal monitor, displays a visual field image created by the image generation unit 43 under control executed by the display control unit 44 (arrow A2).

The information processing unit 30, which comprises an information processing device such as an electronic computer, executes information processing necessary for generation of a visual field image.

It is to be noted that the information processing unit 30, together with the image capturing unit 10 and/or the display unit 20, may configure an integrated unit such as a smart phone, a notebook-type personal computer or the like. Alternatively, the information processing unit 30 may be disposed at a position physically apart from the image capturing unit 10 and/or the display unit 20. In addition, part of the processing executed by the information processing unit 30 may instead be executed by an electronic computer, a server or the like disposed at a remote location, and at least part of the data stored in the information processing unit 30 may instead be stored in a server or the like disposed at a remote location.

The input unit 31 is configured so as to include input devices such as a mouse, a keyboard, various buttons and/or a touch pane or the like. Information and the like needed in processing executed by the control unit 40, entered by the user, are accepted via the input unit 31.

The communication unit 32, configured so as to include a communication device capable of communicating with a network such as the Internet through wireless connection or wired connection, receives prescription data pertaining to the subject S and eyeglass lens shape data, and transmits/receives data as needed.

The storage unit 33, constituted with a nonvolatile storage medium, stores various types of data including the prescription data for the subject S and the eyeglass lens shape data, a program enabling the control unit 40 to execute processing, and the like.

The control unit 40 configured so as to include a processor such as a CPU, executes the program stored in the storage unit 33 as the primary agent of various operations executed in the image generating device 1. The control unit 40 generates a visual field image based upon an image obtained through image capturing operation executed by the image capturing unit 10 and causes the display unit 20 to display it.

The feature detection unit 41 in the control unit 40 detects, through image processing, a feature in an image of the head of the subject S captured by the image capturing unit 10. The term "features" as used in the following description refers to points, lines or portions that can be distinguished from other areas through image processing, such as feature points, edges, corners, two-dimensional and three-dimensional elements that may include such feature points, edges, corners, or the like.

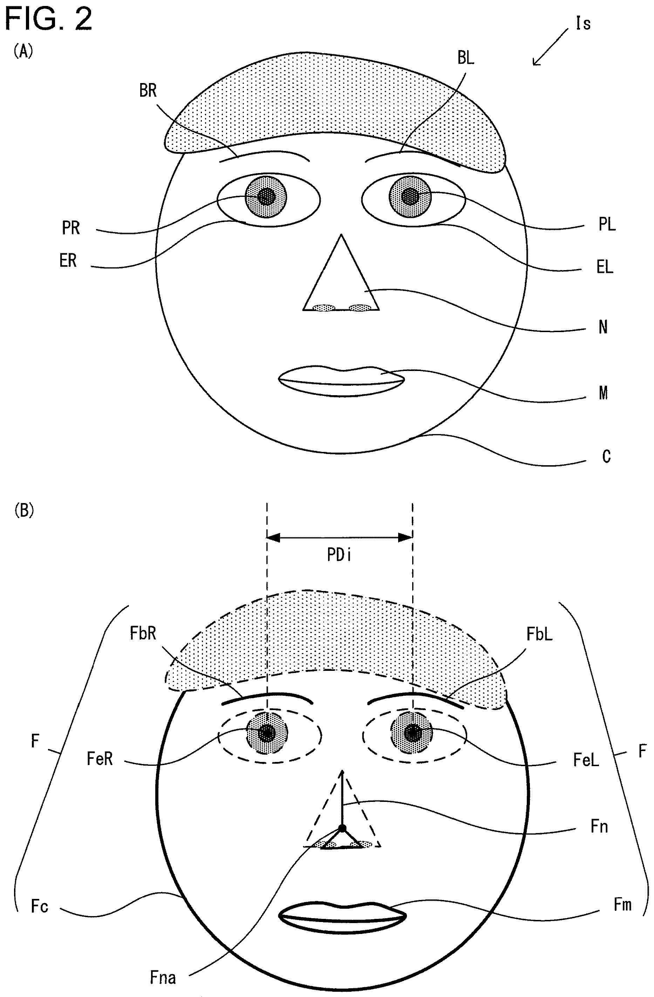

Fig. 2(A) provides a schematic illustration of an image (hereafter referred to as a subject image Is) obtained by capturing an image of the head of the subject S via the image capturing unit 10. The subject image Is in Fig. 2(A) is an image obtained by capturing an image of the subject S from the front and includes parts assuming structural features, colors and the like, such as a left brow BL, a right brow BR, a left eye EL, a right eye ER, a left pupil PL, a right pupil PR, a nose N, a mouth M and a face outline C, in the face of the subject S.

Fig. 2(B) illustrates features F detected by the feature detection unit 41. The feature detection unit 41 detects features F such as feature points, edges and/or corners and the like in the subject image Is by executing image processing through the FAST method, the CANNY method or the like. It is desirable that at least some of the features F be detectable even if the orientation of the face of the subject S relative to the image capturing unit 10 changes. The feature detection unit 41 determines, based upon information pertaining to features F previously obtained and stored in the storage unit 33, as to which part of the face, such as the nose or the face outline C, a detected feature F corresponds to. It is to be noted that the feature detection unit 41 may detect, as features F, a facial feature point detection model obtained through machine learning in conjunction with a face image data set.

Fig. 2(B) shows features F detected from the subject image Is by the feature detection unit 41, including a linear feature FbL corresponding to the left brow BL, a linear feature FbR corresponding to the right brow BR, a feature point FeL corresponding to the center of the pupil in the left eye EL (hereafter referred to as a left-eye feature point), a feature point FeR corresponding to the center of the pupil in the right eye ER (hereafter referred to as a right-eye feature point), a feature point Fna corresponding to the tip of the nose (hereafter referred to as a nose feature point), a feature Fn corresponding to the nose N, which is made up with a line segment extending from the root of the nose to the tip of the nose and line segments branching out from the tip toward the left and right nostrils, a feature Fm corresponding to the outline of the red region of the upper and lower lips M, and a linear feature Fc corresponding to the face outline C.

The feature detection unit 41 stores data indicating the coordinates of the feature points constituting individual features F, data linking the coordinates of a plurality of feature points expressing a line segment constituting each feature F, data indicating the part of the face corresponding to each feature F and the like into the storage unit 33.

It is to be noted that as long as the parameter calculation unit 40 is able to calculate the required parameters as will be explained later, no particular limitations are imposed with respect to the structure of data expressing individual features F.

The parameter calculation unit 42 calculates parameters such as the position, the orientation and the like of the face of the subject S based upon the positions of the features F in the subject image Is detected by the feature detection unit 41. The "face orientation" can be expressed with a roll angle, a pitch angle and a yaw angle of the head, respectively corresponding to the rotational directions schematically illustrated in Fig. 1 and notated with R, P and Y. The roll angle is a rotational angle measured around an axis (z axis) extending to the front/rear relative to the subject S, the pitch angle is a rotational angle measured around an axis (x axis) extending to the left/right relative to the subject S and the yaw angle is a rotational angle measured around an axis (y-axis) extending upward/downward relative to the subject S. In Fig. 1, the directions and the angles of the x-axis, the y-axis and the z-axis are indicated in reference to a coordinate system 900.

It is to be noted that there are no specific limitations to be imposed with respect to the mathematical expression that may be used to indicate the face orientation, including the coordinate system that may be used.

No particular limitations are imposed with respect to the method that may be adopted by the parameter calculation unit 42 when calculating the orientation of the face based upon features F. For instance, a paper authored by Horprasert et. al. (T. Horprasert, Y. Yacoob and L. Davis, "Computing 3-D Head Orientation from a Monocular Image Sequence", Proceedings of the Second International Conference on Automatic Face and Gesture Recognition (US), IEEE, 1996, pp 242 ~ 247) discloses a method for calculating the roll angle, the pitch angle and the yaw angle of the head based upon several feature points including the edges of the eyes (the inner and outer corners of the eyes) and the tip of the nose. In this method, the angles are calculated by assuming that the inner corner and the outer corner of an eye are on a single straight line. The orientation of the face of the subject S may be calculated by the parameter calculation unit 42 by using the subject image Is through any of various head pose estimation methods, instead of a method through which the face orientation is calculated by assuming that feature points corresponding to parts of the face are in a specific spatial relationship, as in the case with the method described in the paper.

The parameter calculation unit 42 is able to calculate the position of the subject S based upon the positions of the features F in the subject image Is detected by the feature detection unit 41, the orientation of the face calculated by the parameter calculation unit 42 and measured values obtained by actually measuring the lengths of parts of the face of the subject S. The parameter calculation unit 42 calculates a distance PDi between the left-eye feature point FeL and the right-eye feature point FeR in the subject image Is based upon the coordinates of these feature points. The parameter calculation unit 42 converts the distance PDi on the image calculated to a length of the distance viewed from the front of the face based upon the orientation of the face calculated and then calculates the distance between the subject S and the image capturing unit 10 based upon a comparison of the converted length and the pupillary distance (PD) obtained through actual measurement.

For instance, the parameter calculation unit 42 is able to determine a distance from the image capturing unit 10 to the subject S, at which the known pupilary distance PD matches the distance PDi on the image, based upon the longitudinal and the lateral dimensions of the focal plane and the focal length of the image sensor in the image capturing unit 10.

It is to be noted that provided that the image generating device 1 comprises a ToF (time of flight) camera capable of capturing a range image by carrying out a range-finding operation through the ToF method or a stereo camera, the distance from the image capturing unit 10 to the subject S may be measured through a method other than that using features F detected in the subject image Is.

As shown in Fig. 1, the parameter calculation unit 42 calculates the distance between the subject S and the display unit 20 as the sum of the distance Ez between the subject S and the image capturing unit 10 calculated and a predetermined distance Cz between the image capturing unit 10 and the display unit 20. In addition, the parameter calculation unit 42 calculates a distance Ey between an eye E of the subject S and the image capturing unit 10, measured along the y-axis, based upon the distance Ez between the subject S and the image capturing unit 10 and the direction in which the eye E of the subject S is seen from the image capturing unit 10. The parameter calculation unit 42 then calculates the position of the eye E along the y-axis in reference to the display unit 20 based upon the distance Ey and a predetermined distance Cy between a position O taken by the display unit 20 and the image capturing unit 10 measured along the y-axis. The parameter calculation unit 42 calculates a three-dimensional position of the eye E in reference to the display unit 20 by executing a calculation in correspondence to the x-axis similar to that executed in correspondence to the y-axis. The distances Cy, Cz and the like that indicate the positional relationship between the image capturing unit 10 and the display unit 20 may be entered by the user via the input unit 31, or numerical values stored in advance in the storage unit 33 may be used as the distances Cy, Cz and the like.

It is to be noted that if the direction along which an image is captured by the image capturing unit 10 is different from the direction along which the normal vector of the display screen of the display unit 20 extends, the angle formed by the image capturing direction and the direction along which the normal vector extends is measured in advance and the measured angle is entered by the user of the image generating device 1 (hereafter referred to as the "user") or the like. Based upon the angle entered as described above, the parameter calculation unit 42 is able to modify the values indicating the three-dimensional positions of the face and the eyes of the subject S and the orientation of the face of the subject S, calculated by using the subject image Is, to values taken in reference to the display unit 20.

Based upon the distance between the subject S and the display unit 20 and the orientation of the face of the subject S, calculated by the parameter calculation unit 42, the image generation unit 43 generates an image of a virtual visual field (visual field image) to be viewed by the subject S through an eyeglass lens. As will be explained later, a visual field image is an image of a visual field that would be viewed when, assuming that a virtual object is present at the position of the display unit 20, the subject S looks at the virtual object through a virtual eyeglass lens from various positions relative to the display unit 20 (i.e., relative to the virtual object). While the visual field image is constructed as a real-time video image with the position and the orientation of the eyeglass lens relative to the visual field image tracking the orientation and the like of the face of the subject S, it may instead be constructed by reproducing a recorded video image that includes a visual field image.

The image generation unit 43 constructs a virtual space by obtaining data indicating positions, shapes, textures and the like of various objects present in the virtual space (hereafter referred to as virtual space description data). Various values included in the virtual space description data are set based upon information stored in the storage unit 33 or information obtained from an external source via the communication unit 32 or based upon input by the user. While no particular limitations are imposed with respect to the mode of the positional arrangement with which objects are set in the virtual space, it is desirable to set a virtual object at the position of the display unit 20 as in the embodiment, since the position and the size of such a virtual object can be more easily ascertained by the subject S looking at the display unit 20. Furthermore, setting a virtual object at the position of the display unit 20 is also desirable since the subject S will be able to ascertain how another virtual object in the virtual space, displayed on the display unit 20, appears in reference to the virtual object set at the position of the display unit 20.

While no particular limitations are imposed with respect to the method through which the virtual space is described with the virtual space description data, the data may include position information indicating the specific position at which, and orientation with which, each object (geometric object) is set in the virtual space, with information indicating the reflectance and the transmittance of light at a surface of the geometric object, the color and the texture of the geometric object and the like also set as needed. A detailed three-dimensional structure or the like of the geometric object can be expressed by substituting, as needed, the information indicating the texture of a planar surface of the geometric object for the three-dimensional structure data. The virtual space description data may include data pertaining to the lighting of the virtual space. Such data pertaining to lighting will include, as needed, information indicating the position of the light source, the color of the light, the wavelength distribution and the intensity of the light.

The image generation unit 43 obtains data pertaining to an eyeglass lens (hereafter referred to as eyeglass lens description data) to be virtually worn by the subject S while looking at the display unit 20. Various values included in the eyeglass lens description data may be set based upon information stored in the storage unit 33, information obtained from an external source via the communication unit 32 or the prescription data for the subject S and the lens type entered by the user. The eyeglass lens prescription data include shape data and material characteristics data pertaining to the eyeglass lens. The shape data for the eyeglass lens include external shape information indicating the external shape of the eyeglass lens, data indicating the central thickness of the eyeglass lens, shape data indicating the shapes of the two surfaces, i.e., the front surface and the rear surface respectively located on the object side and the eyeball side and the shape of the edge surface of the eyeglass lens, and the like. The material characteristics data for the eyeglass lens include data indicating the refractive index and the like.

It is to be noted that the eyeglass lens description data may include values indicating aberration quantities indicating the extent of aberration when looking through the eyeglass lens. It is desirable that the aberration quantities include aberration quantities pertaining to spherical aberration, astigmatism, comatic aberration or the like. The aberration quantities, which are numerical values calculated in advance through ray tracing based upon the prescription data, the lens shape, the lens material, wear parameters to be described later, the distance to the gazing point and/or the accommodation of the eye, and so forth, are expressed in optimal formats such as vectors, matrices or the like.

The image generation unit 43 sets positions of the subject S wearing the eyeglass lens and the eyeglass lens relative to each other (hereafter referred to as an eyeglass lens position). It is desirable that the eyeglass lens position be set based upon data pertaining to the eyeglass lens position (hereafter referred to as eyeglass lens position data) entered by the user. The eyeglass lens position data include wear parameters pertaining to an eyeglass lens position obtained through actual measurement or the like conducted while the subject S is wearing the eyeglass lens and entered by the user, and these wear parameters will include, for instance, a corneal vertex distance, a pantoscopic angle, a warp angle or the like. The user may enter an instruction to indicate that the eyeglass lens position data are not to be used for the particular subject S and in such a case, the image generation unit 43 will set an eyeglass lens position based upon the positional relationship between the eyeglass lens and an eyeball used as a reference positional relationship when designing the eyeglass lens.

Once the parameter calculation unit 43 calculates the position of the eye E of the subject S and the orientation of the subject's face based upon the subject image Is obtained through an image capturing operation executed by the image capturing unit 10, the image generation unit 43 generates a visual field image that would be viewed as the subject S looks at a virtual object present at the position of the display unit 20 from the position assumed by the eye E. Namely, the image generation unit 43 generates a visual field image of a visual field that would be viewed by the subject S looking at the virtual object through the eyeglass lens based upon the position of the eye E, the orientation of the face, the eyeglass lens position, the eyeglass lens description data and the virtual space description data.

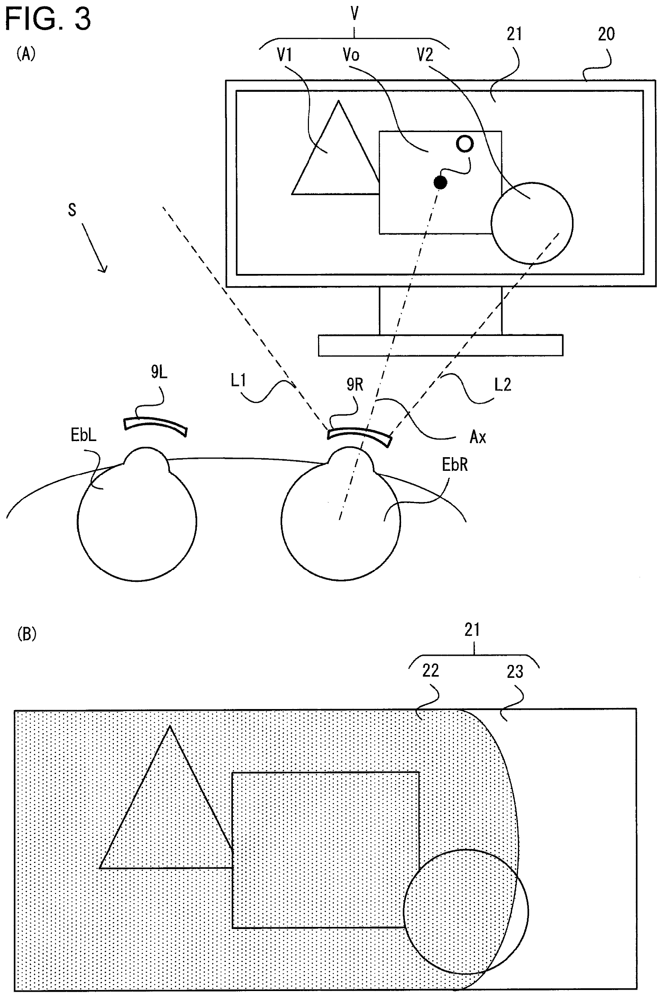

Fig. 3(A) provides a conceptual diagram in reference to which the visual field image will be explained. Eyeglass lenses 9L and 9R in Fig. 3(A) are virtual eyeglass lenses described based on eyeglass lens data. The eyeballs EbL and EbR of the subject S are looking at the display unit 20, and an optical axis Ax of the right eyeball EbR extends through the center O of the display unit 20. At the display unit 20, a visual field image 21 is on display and the visual field image 21 includes images of three virtual objects V (Vo, V1 and V2). The virtual eyeglass lenses 9L and 9R are set at eyeglass lens positions to the front of the left eyeball EbL and the right eyeball EbR.

Dotted lines L1 and L2 in Fig. 3(A) schematically indicate the left end and the right end of the range of the visual field that can be viewed with the right eyeball EbR through the eyeglass lens 9R. In other words, while the left side of the display unit 20 is within the range in which the subject S is able to see it through the eyeglass lens 9R, part of the right side of the display unit 20 is beyond the range in which the subject S is able to see it through the eyeglass lens 9R.

Fig. 3(B) schematically illustrates the visual field image 21 viewed in the situation shown in Fig. 3(A). A hatched part 22 of the visual field image 21 is an image of the visual field viewed by the subject S looking at the display unit 20 through the virtual eyeglass lens 9R. An unhatched part 23 of the visual field image 21 is an image of the visual field that would be viewed by the subject S looking at the display unit 20 without the virtual eyeglass lens 9R. An image of the visual field seen by the subject S with the naked eye is displayed over an area of the visual field image 21 located in the part of the right side of the visual field image 21 that cannot be seen through the eyeglass lens 9R in Fig. 3(A). The image generation unit 43 generates the visual field image 21 based upon the ranges of the eyeglass lenses 9L and 9R in the visual field seen by the subject S as described above.

It is to be noted that the visual field image 21 may be generated based upon the visual field corresponding to either one of the left eye and the right eye, or it may be generated as a composite image by combining visual field images corresponding to the visual fields of the two eyes and locally offsetting the two visual field images based upon, for instance, the extent of dominance of the dominant eye. In addition, while the parameter calculation unit 42 calculates an eye position and the image generation unit 43 generates a visual field image from a viewpoint set at this eye position in this embodiment, it is not necessary to set the viewpoint exactly at an eye position and it may be set at an optimal position such as the halfway point between the two eyes.

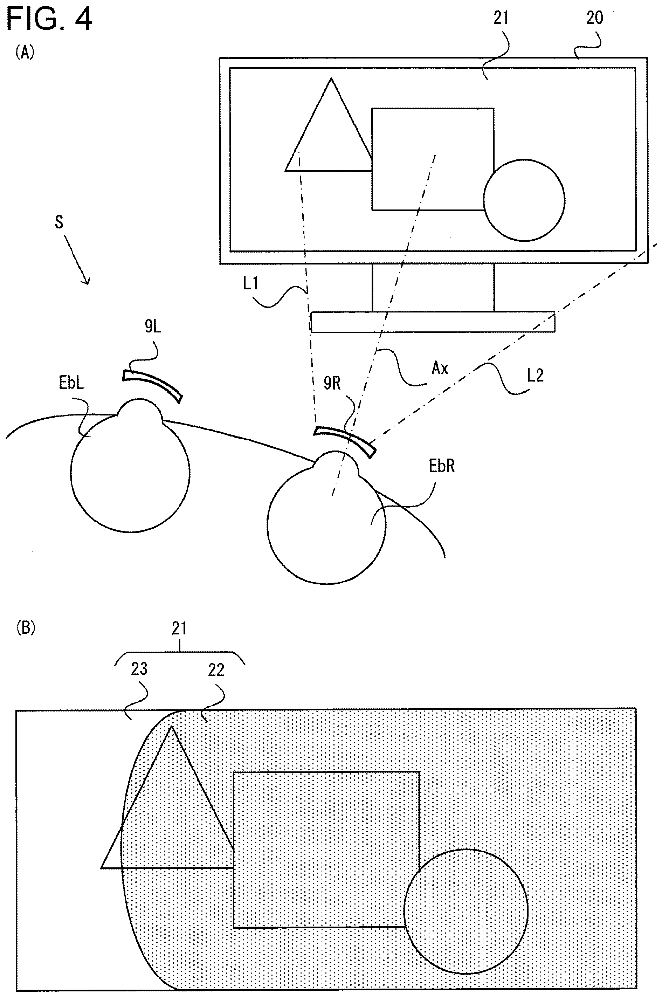

Fig. 4(A) is a conceptual illustration of a state transitioned from the state shown in Fig. 3(A) as the subject S turns his head to the right. In Fig. 4(A) and Fig. 4(B), the same reference signs are assigned to elements identical to those in Fig. 3(A) and Fig. 3(B) and explanation thereof is omitted as appropriate. The left end and the right end of the range of the visual field that can be viewed with the right eyeball EbR through the eyeglass lens 9R, schematically indicated with the dotted lines L1 and L2 respectively, have shifted toward the right side relative to the subject facing opposite the display unit 20. This means that the subject S, looking at the right side of the display unit 20 through the eyeglass lens 9R, cannot see part of the left side of the display unit 20 through the eyeglass lens 9R.

Fig. 4(B) schematically illustrates the visual field image 21 viewed in the situation shown in Fig. 4(A). An image of the visual field seen with the naked eye of the subject S is displayed over an area of the visual field image 21 located in the part of the left side of the visual field image 21 that cannot be seen through the eyeglass lens 9R in Fig. 4(A). If the subject S has altered the eye positions or the orientation of his face, the image generation unit 43 generates a visual field image 21 based upon the changes occurring in the ranges of the eyeglass lenses 9L and 9R in the visual field.

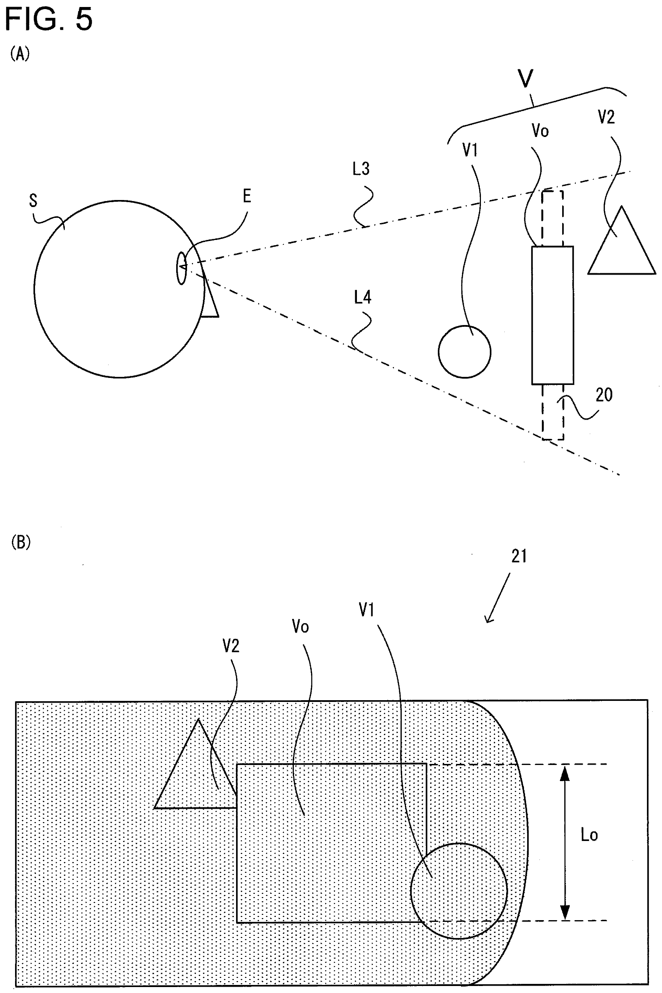

Fig. 5(A) is a conceptual diagram in reference to which adjustment of the angle of view in the visual image 21 will be explained. The virtual space is constructed in the embodiment so that a virtual object (hereafter referred to as a reference object Vo) is set at the position of the display unit 20. When the distance between the subject S and the display unit 20 changes, the size of the reference object Vo in the visual field of the subject S will also change accordingly. However, in order to indicate the relationship among the visual appearances of virtual objects more clearly, the image generation unit 43 adjusts the angle of view for the visual field image 21 so as to allow the reference object Vo to sustain a substantially constant size within the visual field image 21, even under these circumstances.

Fig. 5(A) schematically illustrates virtual objects V including the reference object Vo, the virtual object V1 and the virtual object V2. The virtual space is constructed by assuming that the reference object Vo is located at the position of the display unit 20 (schematically indicated with dotted lines). The visual field of the subject S is schematically indicated as an area present between one-point chain lines L3 and L4 extending from the eye E of the subject S.

Fig. 5(B) shows a visual field image 21 that will come up on display at the display unit 20 in the state shown in Fig. 5(A). Since the virtual object V1, the reference object Vo and the virtual object V2 are set one after another in this order, starting on the side of the subject S as shown in Fig. 5(A), the virtual objects in the visual field image 21 are displayed with overlap so that the virtual object V1 appears to be located at the closest position and the virtual object V2 appears to be located at the furthest position. Since the image generation unit 43 adjusts the angle of view for the visual image 21 so as to allow the reference object Vo to sustain a substantially constant size as explained earlier, the width Lo of the reference object Vo remains substantially constant in the visual field image 21 in spite of the change in the position of the viewpoint.

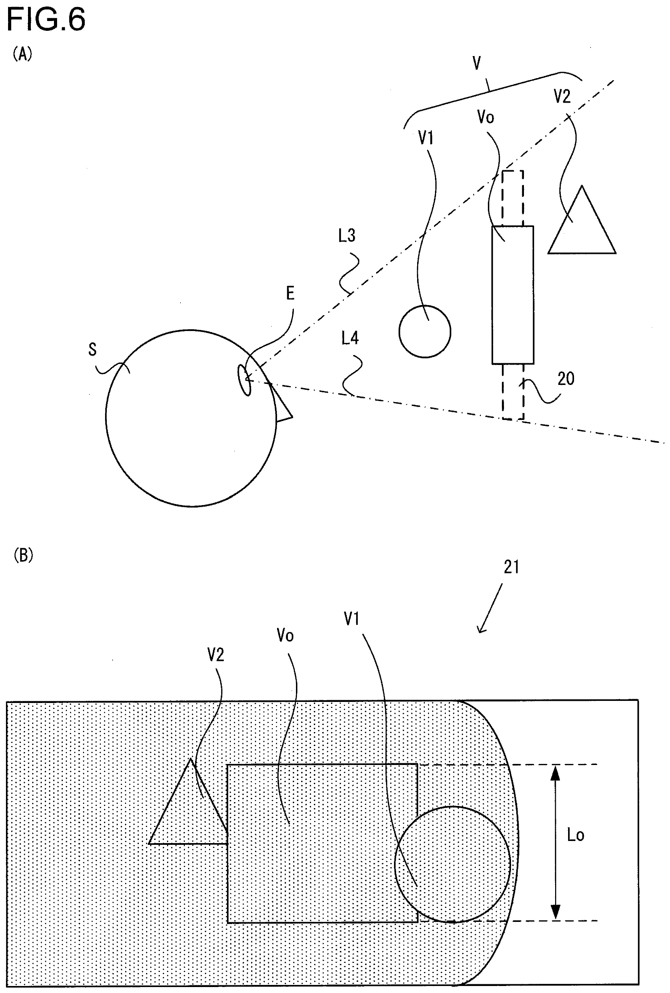

Fig. 6(A) is a conceptual illustration showing a state in which the distance between the subject S and the display unit 20 is smaller than that in Fig. 5(A). In Fig. 6(A) and Fig. 6(B), the same reference signs are assigned to elements identical to those in Fig. 5(A) and Fig. 5(B) and explanation thereof is omitted as appropriate.

Fig. 6(B) shows a visual field image 21 that will come up on display at the display unit 20 in the state shown in Fig. 6(A). Since the distance between the subject S and the reference object Vo is smaller than that in Fig. 5(A), the size of the reference object Vo in the visual field of the subject S will naturally increase. However, adjustment is made so that the reference object Vo sustains a substantially constant size in the visual field image 21, and thus, the width Lo of the reference object Vo in the visual field image 21 also remains substantially constant. In other words, the size of the reference object Vo in the visual field image 21 sustains a constant relation to the size of the display screen of the display unit 20 regardless of the position of the viewpoint.

The image generation unit 43 generating the visual field image 21 generates a projected image by rendering the virtual space through, for instance, perspective projection with the position of an eye of the subject S designated as the viewpoint position. The projected image does not include blurring or skewing, which would be induced by the eyeglass lenses 9L, 9R or the like, as will be explained later, in contrast to the visual field image 21. When generating the projected image, the image generation unit 43, adjusts the angle of view so as to allow the virtual object Vo, set at the position of the display 20, to sustain a substantially constant size regardless of the viewpoint position. In other words, the image generation unit 43 generates the visual field image 21 by ensuring that the angle of view for the visual field image 21 corresponds to the size of the display screen at the display unit 20 regardless of the viewpoint position. As a result, since the appearance of the reference object Vo does not change to any significant degree, changes in the visual appearances of the virtual objects V1 and V2 present around the reference object Vo in the virtual space can be indicated with better clarity in correspondence to changes in the viewpoint position, as indicated in Fig. 5(B) and Fig. 6(B).

Upon generating the projected image, the image generation unit 43 executes image processing so as to add skew attributable to a virtual eyeglass lens 9 (the "eyeglass lens 9" or "eyeglass lenses 9" denotes the eyeglass lens 9L or the eyeglass lens 9R without left-right distinction) to the projected image.

The term "skew" is used in this or the following embodiment to refer to an instance of a target object being perceived as an image of an object taking a shape different from its actual shape, primarily when the image of the target object stretches or contracts along a given direction such as the vertical direction, the horizontal direction or a diagonal direction. While "skew" may include distortion, the skew in this embodiment primarily refers to stretching/contracting of the image of the target object, occurring when the target object is viewed through a surface at which the refractive power or the astigmatism changes, e.g., through the lens surface of a progressive power lens.

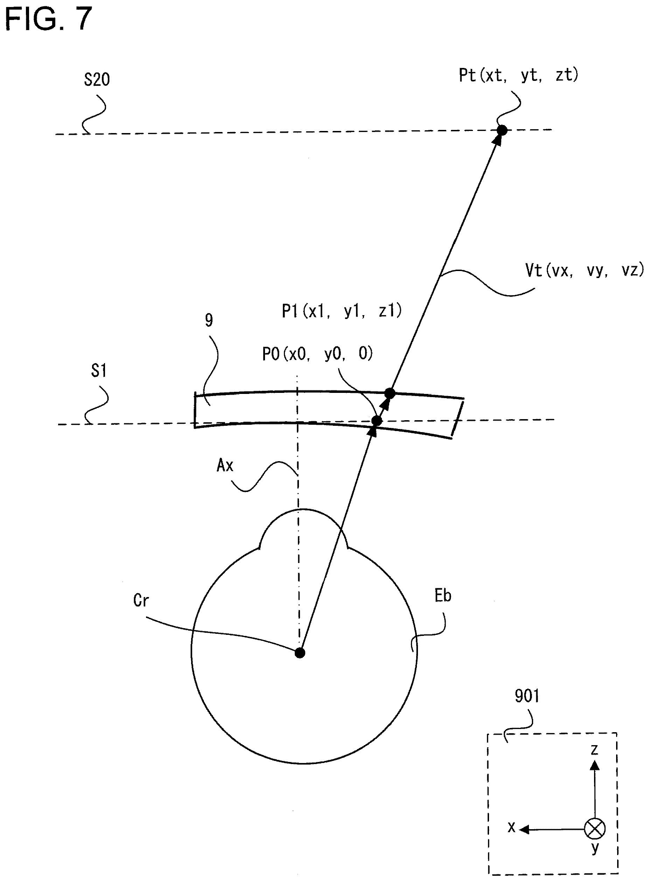

Fig. 7 is a conceptual diagram in reference to which a method of applying skew to the projected image will be explained. The image generation unit 43 calculates an optical path connecting the retina of the subject S looking at the display screen S20 of the display unit 20 with a gazing point Pt on the display screen S20 through ray tracing of a ray traveling from a center of rotation Cr, which is transmitted through the eyeglass lens 9 to pass through a point P1 on the surface of the eyeglass lens 9 and the gazing point Pt. While it is desirable to set the position of the center of rotation Cr by using data obtained through actual measurement conducted with respect to the shape of the face of the subject S, the position of the center of rotation assumed when designing the eyeglass lens 9 or a standard position may be set as the position of the center of rotation Cr.

It is to be noted that the ray tracing calculation may be executed by tracing rays entering various points on the retina or rays exiting various points of the retina.

The image generation unit 43 sets a virtual surface (hereafter referred to as a virtual surface S1) near the eyeglass lens 9. It is desirable that the virtual surface S1 be set so that when the subject S looks straight ahead or the eyeball Eb looks at a fitting point of the eyeglass lens 9, the virtual surface S1 ranges substantially parallel to the display screen S20 or the virtual surface S1 intersects the optical axis Ax of the eyeball Eb substantially perpendicular to the optical axis Ax. The image generation unit 43 traces a ray passing through a point P0 on the virtual surface S1 and adds skew to the projected image based upon the degree of the local skew in the visual field image 21 which is calculated based upon the results of the ray tracing.

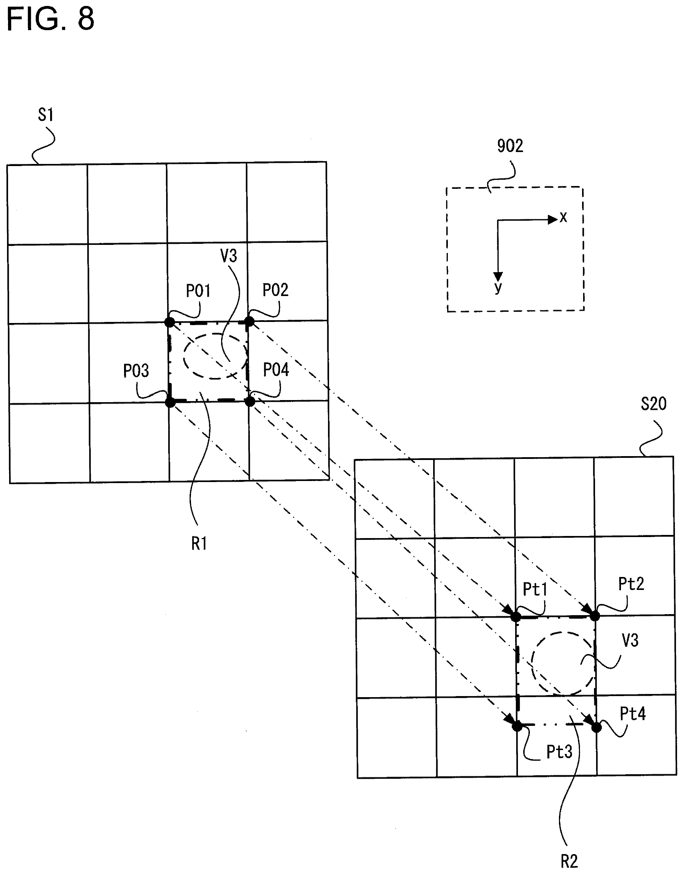

Fig. 8 illustrates a method that may be adopted when calculating the local skew in the visual field image 21 through ray tracing. The virtual surface S1 and the display screen S20 are each shown to be divided with grid lines parallel to the x-axis and the y-axis (see the coordinate system 902) so as to allow the explanation to be clear, and it is assumed that the projected image obtained by rendering the virtual space through perspective projection or the like is set at the position of the display screen S20. The projected image includes a virtual object V3.

The image generation unit 43 calculates, through ray tracing, the positions of corresponding points Pt1, Pt2, Pt3, Pt4 and the like on the display screen S20 at which rays of light, having passed through the center of rotation Cr and coordinate points on the virtual surface S1 such as points P01, P02, P03, P04 and the like, enter the display screen S20.

As shown in Fig. 7, the image generation unit 43 calculates, through ray tracing, a vector Vt which extends from the point P1 on the surface of the eyeglass lens 9, located on the side closer to the display unit 20, toward the point Pt on the display screen S20 at which the ray of light traveling through the point P1 enters. With P1 $(x1, y1, z1)$ indicating the coordinates of the points P1, Pt $(xt, yt, zt)$ indicating the coordinates of the point Pt, Vt $(vx, vy, vz)$ indicating the vector Vt (see the coordinate system 901) and "a" expressed as $(zt - z1)/vz$, the coordinate xt of the point P along the x-axis and the coordinate yt of the point P along the y-axis can be expressed as in (1) below.

$$xt = x1 + a*vx, \; yt = y1 + a*vy \ldots (1)$$

Since the distance between the subject S and the display unit 20 can be substituted for $zt - z1$ in approximation, the image generation unit 43 is able to calculate the x-coordinate xt and the y-coordinate yt of the point P based upon the vector Vt. While the relationship between the point P0 and the point P1 may be determined through ray tracing, the coordinate values for the point P1 can also be used for the x-coordinate value and the y-coordinate value of the point P0 in approximation. The image generation unit 43 is able to set the points P01 through P04 and the points Pt1 through Pt4 (see Fig. 8) in correspondence to each other through this method.

It is to be noted that data pertaining to the eyeglass lens 9 such as the eyeglass lens description data or the eyeglass lens position data may include the vector Vt, which is a parameter linked to the eyeglass lens 9 and indicating the direction of the line of sight from the eyeglass lens 9 to the display unit 20. In such a case, the volume of arithmetic processing to be executed to generate the visual field image 21 can be reduced.

In Fig. 8, a quadrangular partial region (the region inside the two-point chain lines) R1, defined by the points P01, P02, P03 and P04 on the virtual surface S1, corresponds to a partial region R2 defined by the points Pt1, Pt2, Pt3 and Pt4 on the display screen S20. Accordingly, the image generation unit 43 stretches/contracts the projected image so that the portion of the projected image obtained by rendering the virtual space, which corresponds to the partial region R2, is contained in the partial region R1. In the example presented in Fig. 8, since the partial region R2 on the display screen S20 corresponds to the partial region R1 on the virtual surface S1, the width of which measured along the y-axis is smaller than that of the partial region R2, the projected image is processed so that the image of the virtual object V3 corresponding to the partial region R2 is contracted along the y-axis. In other words, a skew determined through linear interpolation is applied with respect to the points on the eyeglass lens 9 for which no ray tracing is executed.

The image generation unit 43 calculates the direction and the extent of local skew in the visual field image 21 based upon the direction along which light having departed the eye of the subject S exits the eyeglass lens 9 as described above, and generates a visual field image 21 that includes the skew. Through this method, the image generation unit 43 is able to generate a visual field image 21 with skew applied thereto without having to trace rays for all the coordinate points corresponding to the surface of the eyeglass lens 9. As a result, the image generation unit 43, capable of generating visual field images 21 with skew applied thereto at high speed, is able to generate a video image constituted with these visual field images 21 at a higher frame rate.

Upon applying skew to the projected image of the virtual space as seen from the viewpoint position, which has been obtained by rendering the virtual space, the image generation unit 43 executes image processing through which blur that would be induced by the virtual eyeglass lens 9 is applied to the projected image.

The term "blur" used in this or the following embodiment refers to the loss of detail of a target object, which occurs when the target object is seen with a resolution lower than the optimal resolution for viewing the target object. In more specific terms, the term "blur" is used to refer to a reduction in the definition of edges and patterns, primarily occurring in an image captured in a defocused state (in an out-of-focus state) or in an image perceived when the image forming plane on which an image is formed with light from the target object, determined by the optical aberration of the ocular optical system, is offset from the retina.

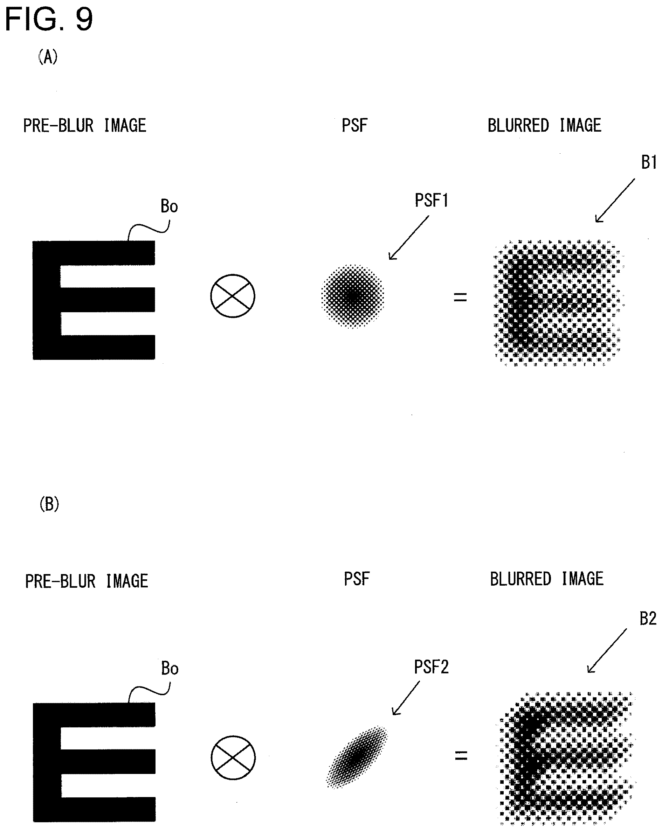

Fig. 9(A) and Fig. 9(B) each present a conceptual diagram of an example of a method through which blur is applied to an image. Fig. 9(A) is a conceptual diagram illustrating image processing executed to create a blurred image B1 without direction dependence by using a pre-blur image Bo. The blurred image B1 in this case may be generated based upon, for instance, blur attributable to a refractive power error occurring when no astigmatism manifests.

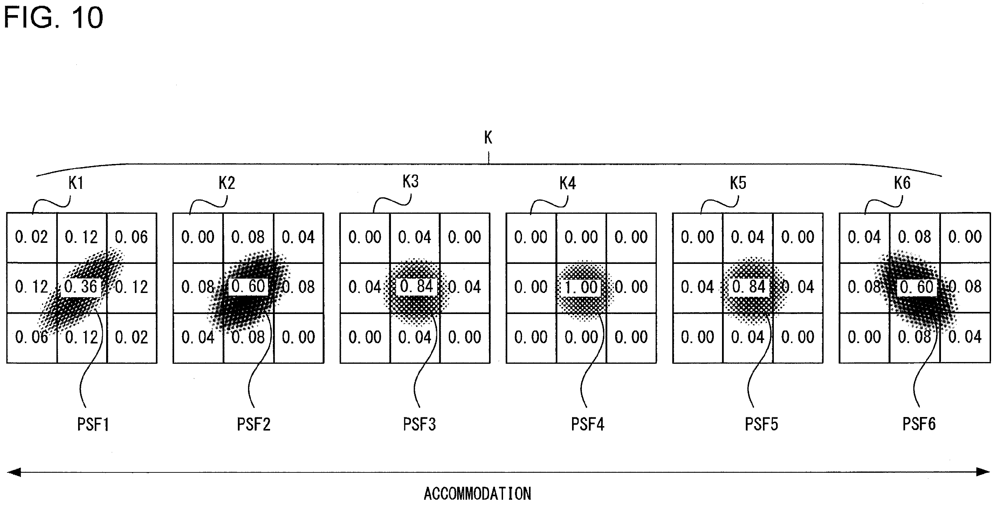

The blurred image B1 can be obtained through convolution of the pixel values at the individual pixels in the image by using a point spread function (PSF) as a kernel. The symbol with X inside a circle indicates convolution. By convolving the pre-blur image Bo with a point-spread function PSF1 with no direction dependence, an image with blur occurring uniformly at the various points, such as the blurred image B 1, is obtained. The blurred image B1 is a blurred image B1 having no direction dependence. The convolution operation is equivalent to convolution processing executed by using a matrix such as 3 x 3 or 5 x 5 (see Fig. 10, hereafter referred to as a blur kernel matrix) as a kernel in a discrete calculation executed on a computer.

The conceptual diagram in Fig. 9(B) illustrates image processing through which a blurred image B2 with direction dependence is generated. In this blurred image B2, blur attributable to astigmatism and a refractive power error is simulated.

By executing convolution in the pre-blur image Bo with a point spread function PSF2 having direction dependence (along a diagonal 45° direction), an image with various points blurred to a greater extent along the diagonal 45° direction, such as the blurred image B2, is obtained. The blurred image B2 is a blurred image B2 with direction dependence.

The blurred images B1 and B2 in the examples presented in Figs. 9(A) and 9(B) are each generated through convolution processing executed by using the same point spread function for the entirety of the pre-blur image Bo. However, the image generation unit 43 is able to generate a visual field image 21 through convolution processing executed by using, as kernels, optimal blur kernel matrices corresponding to different point spread functions at various positions in the projected image.

The image generation unit 43 obtains data pertaining to the refractive power of the subject's eye, such as data indicating the spherical power, the astigmatic power and the astigmatic axis, by referencing the eyeglass lens prescription data for the subject S via the input unit 31 or the communication unit 32, or by referencing the eyeglass lens prescription data included in the data for the subject S stored in the storage unit 33. The image generation unit 43 sets a blur kernel matrix for the subject S that will be optimal when the subject S focuses on the display unit 20 based upon the data pertaining to the refractive power of the subject's eye obtained and the refractive power of the eyeglass lens 9 achieved when looking at a predetermined position, such as the center O (see Fig. 1) of the display unit 20.

The image generation unit 43 obtains information indicating the accommodation ability of the subject S. The accommodation ability of the subject S may be set as, for instance, a numerical value (hereafter referred to as an accommodation parameter) in units of diopters (D) based upon the amplitude of accommodation ability. The accommodation parameter may be entered by the user via the input unit 31, may be set according to the age of the subject S indicated in his prescription data based upon known data, or may be calculated based upon the added power or the like if the prescription data are for a progressive power lens or the like.

The image generation unit 43 sets a blur kernel matrix K by determining an aberration quantity through ray tracing based upon the refractive power calculated for the subject S based upon the data indicating the spherical power, the astigmatic power and astigmatic axis, the refractive power and the astigmatism of the eyeglass lens 9 manifesting when the subject S looks at the center O of the display unit 20 or the like, the distance between the subject S and the display unit 20, the position of an object in the virtual space and the accommodation ability indicated by the accommodation parameter. When the accommodation ability is greater, the extent to which the refractive power of the eye changes, too, is greater, and accordingly, each blur kernel matrix K for every certain degree of accommodation, e.g., 0.25d, is set in correspondence to the extent to which the refractive power changes.

Fig. 10 is a conceptual diagram indicating how the blur kernel matrix K may change as the refractive power changes. As the subject S increases the refractive power of the eyeball Eb through accommodation, the blur kernel matrix K changes in sequence from a blur kernel matrix K1 to blur kernel matrices K2, K3, K4, K5, and K6 in this order. Fig. 10, presenting an example in which the blur kernel matrix K is a 3 x 3 matrix, schematically illustrates the concept by superimposing corresponding point spread functions PSF1, PSF2, PSF3, PSF4, PSF5 and PSF6 over the blur kernel matrices K1, K2, K3, K4, K5, and K6 respectively.

The blur kernel matrix K4 with the numerical value of 1.00 taken for the center is a kernel without any blur. It is assumed that the subject S adjusts the refractive power so as to minimize blur at the gazing point within the accommodation ability. Accordingly, the image generation unit 43 sets the blur kernel matrix K4 with the least blur, i.e., the blur kernel matrix achieving the greatest numerical value for the central element in the blur kernel matrix K, among the blur kernel matrices K corresponding to the refractive powers that can be achieved based upon the accommodation ability of the subject S, as the kernel at the position in the visual field image 21 corresponding to the center O of the display unit 20. The image generation unit 43 obtains the value representing the refractive power of the subject S corresponding to this blur kernel matrix K4 (hereafter referred to as an accommodated refractive power).

If there are a plurality of blur kernel matrices K with the greatest value taken as numerical values at the central elements thereof, among all conceivable blur kernel matrices K, the image generation unit 43 sets the blur kernel matrix K corresponding to the least extent of accommodation as the kernel for the position in the visual field image 21 corresponding to the center O of the display unit 20.

The image generation unit 43 calculates a blur kernel matrix K for each point in the visual field image 21 based upon the accommodated refractive power obtained, the astigmatic power and the astigmatic axis, the position of each point within the virtual space, and the refractive power and the astigmatism at the position of the eyeglass lens 9 through which the line of sight, when looking at each point mentioned above, passes.

It is to be noted that a blur kernel matrix K may be calculated for the portion of the visual field image 21 that is viewed without the eyeglass lens 9 based upon the accommodation ability of the subject S obtained as has been explained earlier, or blur may be applied to the portion of the visual field image 21 based upon optometric data obtained by examining the naked eye of the subject S.

The image generation unit 43 traces light rays departing various points on a virtual object V set in the virtual space, which are transmitted through the eyeglass lens 9 and then enter the retina of the eye E. During this process, the image generation unit 43 sets lens parameters in an eyeball model based upon the adjusted accommodation ability ascertained as described earlier. For these purposes, an eyeball model constructed based upon eyeball data obtained through measurement in the past may be utilized as appropriate. The image generation unit 43 is able to calculate an aberration quantity representing the extent of aberration at each point in the visual field image 21 based upon the position at which the various light rays, entering various points on the retina in the ray tracing, converge. The image generation unit 43 sets the blur kernel matrix K for each point based upon the aberration quantity at each point mentioned above in the visual field image 21.

It is to be noted that the image generation unit 43 may obtain data linking blur kernel matrices K, calculated in advance, by tracing rays in correspondence to various points on the eyeglass lens 9 based upon the eyeglass lens shape data, the eyeglass lens position data and the like, to the eyeglass lens.

Once the blur kernel matrix K corresponding to each point in the visual field image 21 is set, the image generation unit 43 generates the visual field image 21 through convolution processing executed for matrices indicating pixel values in the image to which skew is applied as explained earlier by using the blur kernel matrices K as kernels.

It is to be noted that blur may be first applied as described above to a two-dimensional image obtained through rendering of the virtual space based upon the viewpoint position by using the blur kernel matrices K and then skew may then be applied.

The display control unit 44 (see Fig. 1) executes control on display of the visual field image 21, generated by the image generation unit 43, at the display screen of the display unit 20. The display control unit 44 brings up on display in sequence, visual field images 21, sequentially generated by the image generation unit 43 based upon subject images Is sequentially captured by the image capturing unit 10, at the display unit 20.

The subject S, looking at the visual field image 21 on display at the display unit 20 from various positions, is able to perceive how the reference object Vo or the like located at the position of the display unit 20 would appear through the virtual eyeglass lens 9 in a simulated experience. The image generating device 1 can be configured as an eyeglass lens selection system that enables the subject S to select the optimal eyeglass lens to wear based upon the simulated experience.

In addition, the subject S is able to perceive, as a simulated experience, how other virtual objects V1 and V2 located around the reference object Vo in the virtual space would appear in the visual field through the virtual eyeglass lens 9. An eyeglass lens wearer may be particular about how an object appears at a point away from the gazing point through a progressive power lens or about how an object appears through the marginal portion of a mono-focal lens. The image generation device 1 in the embodiment is particularly ideal in applications in which such appearances of objects viewed through eyeglass lenses are simulated.

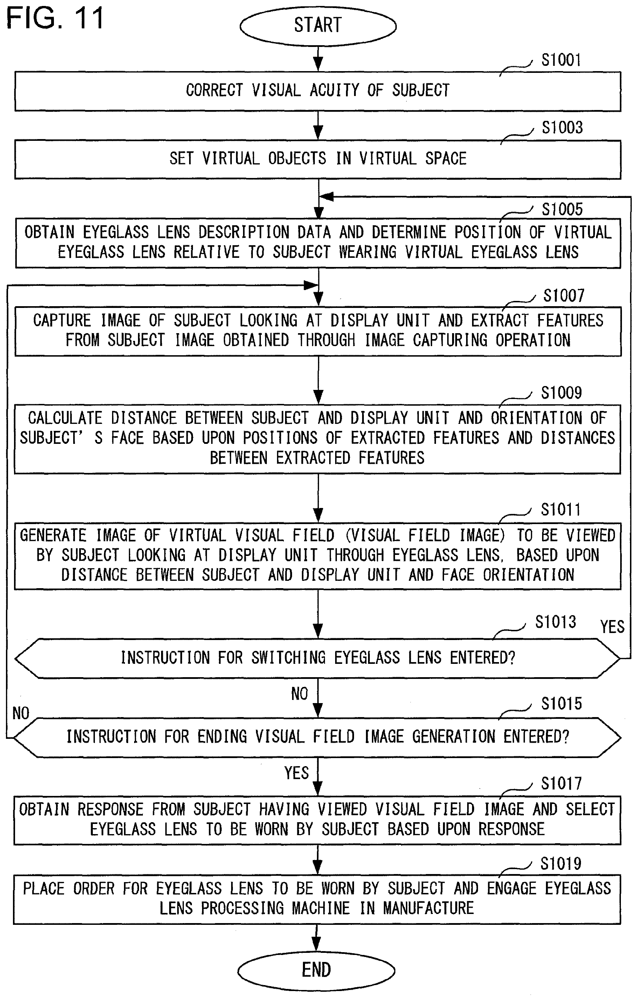

Fig. 11 presents a flowchart of the flow of a method for image generation in which the image generating device according to the embodiment is used. In step S1001, the visual acuity of the subject S is corrected through, for instance, the use of corrective lenses so as to allow the subject S to view an image to be brought up on display at the display unit 20 with ease. The corrective lenses may be eyeglass lenses worn by the subject S in daily life. Once step S1001 ends, step S1003 starts. In step S1003, the image generation unit 43 constructs a virtual space by setting virtual objects V in the virtual space. Once step S1003 ends, step S1005 starts.

In step S1005, the image generation unit 43 obtains eyeglass lens description data and determines the position of a virtual eyeglass lens 9 worn by the subject S relative to the subject S. Once step S1005 ends, step S1007 starts.

It is to be noted that steps S1001, S1003 and S1005 may be executed in a different order.

In step S1007, the image capturing unit 10 captures an image of the subject S looking at the display unit 20 and the feature detection unit 41 detects features F in a subject image Is obtained through the image capturing operation. Once step S1007 ends, step S1009 starts. In step S1009, the parameter calculation unit 42 calculates the distance between the subject S and the display unit 20 and the orientation of the face of the subject S based upon the positions of the detected features F and distances between the detected features F. Once step S1009 ends, step S1011 starts.

In step S1011, the image generation unit 43 generates an image of a virtual visual field (visual field image 21) that would be viewed by the subject S looking at the display unit 20 through an eyeglass lens 9, based upon the distance between the subject S and the display unit 20 and the orientation of the subject's face. Once step S1011 ends, the operation proceeds to step S1013.

In step S1013, the control unit 40 makes a decision as to whether or not the user has entered a switch instruction for switching to a different eyeglass lens 9. If a switch instruction has been entered, the control unit 40 makes an affirmative decision in step S1013 and the operation returns to step S1005. If no switch instruction has been entered, the control unit 40 makes a negative decision in step S1013 and the operation proceeds to step S1015.

In step S1015, the control unit 40 makes a decision as to whether or not an end instruction for ending generation of a visual field image 21 has been entered. If an end instruction has been entered, the control unit 40 makes an affirmative decision in step S1015 and the operation proceeds to step S1017. If, on the other hand, no end instruction has been entered, the control unit 40 makes a negative decision in step S1015 and the operation returns to step S1007. Until an end instruction is entered, steps S1007 through S1015 are repeatedly executed and thus, visual field images 21 are sequentially displayed as a video image.

In step S1017, the user ascertains a response of the subject S having viewed the visual field image 21 and an eyeglass lens to be worn by the subject S is selected based upon the response. For instance, the subject S, visiting an optometric shop, may look at the visual field image 21 virtually viewed through an eyeglass lens he is thinking about purchasing. This visual field image 21 is an image viewed through a virtual eyeglass lens having the spherical power, the astigmatic power, the additional power and the like determined based upon prescription data obtained in advance for the subject S, and the visual field image 21 is thus a virtual image that would be viewed by the subject looking at the display unit 20 with visual acuity corrected through the eyeglass lens. The user, i.e., a sales clerk in the optometric shop, monitors a visual impression experienced by the subject S looking at the visual field image 21. Based upon the visual impression, an eyeglass lens to be purchased by the subject S is selected and an order is entered at an eyeglass lens order-placing device that is not shown in the figures.

It is to be noted that information pertaining to the eyeglass lens to be worn by the subject S may be entered at the image generating device 1 and an order may be placed via the image generating device 1. In addition, the subject S, having looked at the visual field image 21 may enter information specifying the eyeglass lens he wishes to wear (purchase) via the input unit 21 for himself. Once step S1017 ends, step S1019 starts.

In step S1019, the eyeglass lens order-placing device places an order for the eyeglass lens to be worn by the subject S, and an eyeglass lens order-accepting device (not shown in the figures) having received the order for the eyeglass lens engages an eyeglass lens processing machine (not shown in the figures) in the manufacture of the eyeglass lens. Once step S1019 ends, the processing ends.

The following advantages and operations are achieved through the embodiment described above.

(1) The image generating device achieved in the embodiment comprises a feature detection unit 41 that detects features F in a subject image Is obtained by capturing an image of a subject S looking at a display unit 20, a parameter calculation unit 43 that calculates a distance between the subject S and the display unit 20 and an orientation of the face of the subject S based upon the positions of the features F having been detected and/or distances between the detected features F, an image generation unit 43 that generates a visual field image 21, which is to be viewed by the subject S looking at the display unit 20 through an eyeglass lens 9 based upon the distance between the subject S and the display unit 20 and the orientation of the subject's face, and a display control unit 44 that causes the display unit 20 to display the visual field image 21. This image generating device makes it possible for the subject S to have a simulated experience of looking at an object through the eyeglass lens 9 through efficient processing using the features F in an image generated by capturing an image of the subject S. In addition, it provides an experience closely approximating the actual experience of visually perceiving an object through the eyeglass lens 9, in conjunction with an image captured with a standard camera without having to use a special device such as a range image sensor or a head-mounted display unit.

(2) In the image generating device achieved in the embodiment, the parameter calculation unit 42 calculates the position of an eye of the subject S based upon the positions of the detected features F and/or the distances between the detected features F, and the image generation unit 43 generates a visual field image 21 to be viewed by the subject S looking at the display unit 20 from the eye position through the eyeglass lens 9 based upon the eye position, the distance between the subject S and the display unit 20 and the orientation of the subject's face. This structure makes it possible to determine a viewpoint position with better precision and thus, the subject S is able to have an experience that even better approximates the actual visual appearance of an object seen through the eyeglass lens 9.

(3) The image generation unit 43 in the image generating device achieved in the embodiment generates a visual field image 21 to be viewed by the subject S looking at a virtual object (reference object Vo) located at the position taken by the display unit 20. Via this image, the subject S is able to have an experience effectively simulating how an object located at the position of the display unit 20 would appear.

(4) The image generation unit 43 in the image generating device achieved in the embodiment constructs a three-dimensional virtual space that contains the reference object Vo and sets the angle of view of the visual field image 21 in correspondence to the size of the display screen S20 at the display unit 20 regardless of the eye position of the subject S. This means that since another virtual object V is displayed in reference to the reference object Vo the size and the position of which can be ascertained with ease, the subject S is able to perceive a convincing stereoscopic perspective and is also able to perceive how an object appears at a position other than the gazing point.

(5) The image generation unit 43 in the image generating device achieved in the embodiment calculates a local skew attributable to the eyeglass lens 9 based upon the direction along which light, having departed the eye E, exits the eyeglass lens 9. Through processing executed on various portions of the visual field image 21 by applying such local skew, the visual field image 21 can be generated at high speed.

(6) The image generation unit 43 in the image processing device achieved in the embodiment calculates an aberration quantity indicating extent of aberration occurring at the retina based upon a result obtained by tracing rays of light having departed points on a virtual object V set in the virtual space, transmitted through the eyeglass lens 9 and entering the retina of the eye E, and generates a visual field image 21 that includes blur through a convolution operation executed based upon the aberration quantity. This allows the subject S to experience a visual perception of looking through the eyeglass lens 9 that closely approximates reality by simulating blur that would occur in correspondence to various points on the eyeglass lens 9.

(7) The image processing device achieved in the embodiment calculates an aberration quantity based upon, for instance, parameters determined based on the accommodation ability and the age of the subject S and/or prescription data for the subject S. As a result, since the accommodation ability of the subject S is factored in, the subject S is able to experience a visual perception better approximating the reality of looking through the eyeglass lens 9.

(8) The image generation unit 43 in the image processing device achieved in the embodiment alters the blur kernel matrix used as the kernel in the convolution operation based upon the distance between the subject S and the display unit 20. Thus, a blur kernel matrix can be set through a lighter volume of processing and blur can be added to the visual field image 21 at high speed.

(9) The parameter calculation unit 42 in the image processing device achieved in the embodiment calculates the position and the attitude of the eyeglass lens 9 assumed at the time of generation of the visual field image 21, based upon the prescription data for the subject S and/or values obtained through actual measurement conducted on the subject S wearing the eyeglass lens. Thus, the subject S is able to have an experience closely approximating the actual visual perception corresponding to the eyeglass lens position optimized for the subject S.

(10) The feature detection unit 41 in the image processing device achieved in the embodiment detects features F in a portion of the subject image Is that corresponds to the face of the subject S. Since this makes it possible to calculate parameters such as the distance between the subject S and the display unit 20 in conjunction with an image obtained by capturing an image of the face of the subject S, highly efficient processing is enabled. In addition, since a device with a lens positioned to the front of the eye of the subject, such as a head-mounted display unit, is not required, the need for taking into consideration aberration attributable to such a lens is eliminated.

(11) In the image processing method achieved in the embodiment, a plurality of features are detected in a subject image Is obtained by capturing an image of a subject S looking at a display unit 20, the distance between the subject S and the display unit 20 and the orientation of the face of the subject S are calculated based upon the positions of the features F having been detected and/or distances between the detected features F, a visual field image 21 that is to be viewed by the subject S looking at the display unit 20 through an eyeglass lens 9 is generated based upon the distance between the subject S and the display unit 20 and the orientation of the subject's face, and the visual field image 21 is brought up on display at the display unit 20. Through this method, the subject S is allowed to have an experience simulating a visual perception that would be experienced through the eyeglass lens 9 through efficient processing executed in conjunction with the features F in an image obtained by capturing an image of the subject S.

The following variations are also within the scope of the present invention and may be adopted in combination with the above embodiment. Elements assigned with the same reference signs as those in the above embodiment achieve identical functions and explanation thereof will not be provided as appropriate.

Variation 1

In the embodiment described above, the parameter calculation unit 42 calculates parameters such as the distance between the subject S and the display unit 20 by using the pupillary distance PD from features F pertaining to the subject S. As an alternative, the subject S may wear around his head a member having a portion with a known length, the parameter calculation unit 42 may calculate parameters such as the distance between the subject S and the display unit 20 in reference to the length of the portion in the subject image 1s. For instance, a mark or the like, that can be detected by the feature detection unit 41 as a feature F may be added to a corrective lens worn by the subject S when he looks at the display unit 20 and/or a frame of the corrective lens or the like.

As described above, a structure in which the feature detection unit 41 detects a feature F from a portion of the subject image Is that corresponds to a member set around the head of the subject S may be adopted. This structure enables accurate calculation of parameters such as the distance between the subject S and the display 20 based upon a feature that can be detected with ease.

Variation 2

While the image generating device 1 in the above embodiment comprises a single display unit 20, it may instead comprise a plurality of display units 20. In such a case, it is desirable that the plurality of display units 20 be disposed at varying distances from the subject S. For instance, it is desirable that the plurality of display units 20 be disposed at least at two different distances from the subject S, selected from a long distance (set at an optimal value, e.g., 1 m or greater), a short distance (set at an optimal value, e.g., less than 50 cm), and an intermediate distance less than the long distance and greater than the short distance. This configuration allows the subject S to have an experience even more closely simulating the visual perception achieved through the virtual eyeglass lens 9 by simulating, for instance the visual appearances of objects present at different distances, seen as the line of sight of the subject S moves.

Variation 3

The display unit 20 in the embodiment described above may include a three-dimensional display unit. In such a case, the image generation unit 43 generates a visual field image 21 with the position of the left eye of the subject S set as a viewpoint, which is to be used as a left-eye visual field image, and a visual field image 21 with the position of the right eye of the subject set as a viewpoint, which is to be used as a right-eye visual field image. When the subject S looks at the three-dimensional display unit in the display unit 20, he wears eyeglasses with special optical characteristics or a parallax barrier is used so that the left-eye visual field image is seen by the left eye of the subject S and the right-eye visual field image is seen by the right eye of the subject S. As a result, the subject S is able to perceive the visual field image 21 as a stereoscopic space and thus is able to have an experience even more closely approximating reality.

Variation 4

While the image generating device in the embodiment described above comprises a single image capturing unit 10, it may instead comprise a plurality of image capturing units 10. By using subject images Is obtained via the plurality of image capturing units 10, the distance between the subject S and the display unit 20 and the orientation of the face of the subject S can be measured with even higher accuracy.

Variation 5

A program that enables the image generating device 1 to fulfill information processing functions may be recorded into a computer-readable recording medium and a program recorded in the recording medium, pertaining to control under which the image generation processing described earlier and related processing are executed, may be read into a computer system so that the processing is executed in the computer system. It is to be noted that the term "computer system" in this context may refer to an OS (operating system) or a peripheral device in hardware. In addition, the "computer-readable recording medium" may be a portable recording medium such as a flexible disk, a magneto-optical disk, an optical disk or a memory card, or it may be a storage device such as a hard disk built into the computer system. Furthermore, the "computer-readable recording medium" may be a medium that dynamically holds the program over a short period of time, e.g., a communication line through which the program is transmitted via a network such as the Internet or via a communication network such as a telephone network, or a medium that holds the program over a certain length of time, e.g., a volatile memory within a computer system functioning as a server or a client in the above case.

Moreover, the program may allow only some of the functions described above to be fulfilled or the functions described above may be fulfilled by using the program in conjunction with a program pre-installed in the computer system.

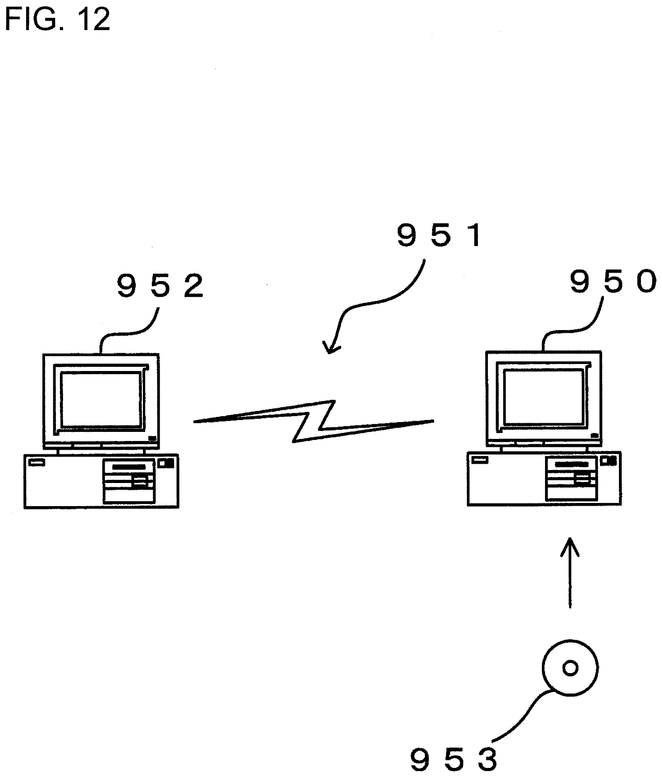

In addition, the present invention may be adopted in conjunction with a personal computer (hereafter referred to as a PC) or the like, and in such a case, the program pertaining to the control described above can be provided in a recording medium such as a CD-ROM or DVD-ROM or on a data signal transmitted through the Internet or the like. Fig. 12 illustrates how such a program may be provided. A PC 950 receives the program via a CD-ROM 953. The PC 950 is also capable of connecting with a communication network 951. A computer 952 is a server computer that provides the program stored in a recording medium such as a hard disk. The communication network 951 may be a communication network such as the Internet or a personal computer communication network, or it may be a dedicated communication network. The computer 952 reads out the program from the hard disk and transmits it to the PC 950 via the communication network 951. In other words, the program may be delivered as a data signal carried on a carrier wave transmitted via the communication network 951. Namely, the program can be distributed as a computer-readable computer program product assuming any of various modes including a recording medium and a carrier wave.

The program enabling the information processing functions to be fulfilled described above may be a program that enables a processing device to execute: feature detection processing through which a plurality of features F are detected from a subject image Is obtained by capturing an image of a subject S looking at a display unit 20, parameter calculation processing through which the distance between the subject S and the display unit 20 and the orientation of the face of the subject S are calculated based upon the positions of the features F having been detected and/or a distance between the detected features F, image generation processing through which a visual field image 20 that is to be viewed by the subject S looking at the display unit 20 through an eyeglass lens 9 is generated based upon the distance between the subject S and the display unit 20 and the orientation of the face, and display control processing through which the visual field image 21 is displayed at the display unit 20.

The present invention is in no way limited to the particulars of the embodiment described above. More specifically, the matters described in reference to the above embodiment and the variations thereof may be adopted in combination as deemed appropriate. Other modes conceivable within the scope of the technical teaching of the present invention are also within the scope of the present invention.

REFERENCE SIGNS LIST

1... image generating device, 9, 9L, 9R... eyeglass lens, 10... image capturing unit, 20... display unit, 21... visual field image, 30... information processing unit, 40... control unit, 41... feature detection unit, 42... parameter calculation unit, 43... image generation unit, 44... display control unit, E... eye, EbL, EbR... eyeball, F... feature, K, K1, K2, K3, K4, K5, K6... blur kernel matrix, S... subject, S1... virtual surface, S20... display screen, Vo... reference object,

**Claims**

1. An image generating device (1), comprising:

   a feature detection unit (41) that detects features (F) in a captured image (Is) obtained by capturing an image of a subject looking at a display device (20);
   a parameter calculation unit (42) that calculates a distance between the subject and the display device and an orientation of a face of the subject based upon positions of the features having been detected and/or a distance between the features having been detected;
   an image generation unit (43) that generates an image of a virtual visual field, which is a visual field as would be viewed by the subject looking at the display device through a virtual eyeglass lens (9), based upon virtual eyeglass lens data, the distance between the subject and the display device and the orientation of the face; and
   a display control unit (44) that causes the display device to display the image of the virtual visual field.

2. The image generating device according to claim 1, wherein:

   the parameter calculation unit calculates an eye position with respect to an eye of the subject based upon the positions of the features having been detected and/or the distance between the features having been detected; and
   the image generation unit generates an image of a virtual visual field to be viewed by the subject looking at the display device through the virtual eyeglass lens from the eye position, based upon the eye position, the distance between the subject and the display device and the orientation of the face.

3. The image generating device according to claim 2, wherein:
   the image generation unit generates the image of the virtual visual field to be viewed by the subject looking at a virtual object located at a position taken by the display device.

4. The image generating device according to claim 3, wherein:
the image generation unit constructs a three-dimensional virtual space that contains the object and adjusts an angle of view of the image of the virtual visual field in correspondence to a size of a display screen of the display device regardless of the eye position.

5. The image generating device according to claim 4, wherein:
the image generation unit calculates a local skew attributable to the virtual eyeglass lens based upon a direction along which light having departed the eye exits the eyeglass lens.

6. The image generating device according to claim 4 or claim 5, wherein:
the image generation unit calculates an aberration quantity indicating extent of aberration occurring at a retina of the eye based upon a result obtained by tracing rays of light, having departed points on the object set in the virtual space, transmitted through the eyeglass lens and entering the retina, and generates the image of the virtual visual field that is blurred through a convolution operation executed based upon the aberration quantity.

7. The image generating device according to claim 6, wherein:
the image generation unit calculates the aberration quantity based upon at least one of accommodation ability of the subject, an age of the subject and prescription data pertaining to the subject.

8. The image generating device according to claim 6 or claim 7, wherein:
the image generation unit alters a kernel of the convolution operation based upon the distance between the subject and the display device.

9. The image generating device according to any one of claims 1 through 8, wherein:
the parameter calculation unit calculates a position and an attitude of the virtual eyeglass lens while generating the image of the virtual visual field based upon prescription data pertaining to the subject and/or measurement values obtained through actual measurement conducted on the subject wearing the eyeglass lens.

10. The image generating device according to any one of claims 1 through 9, wherein:
the feature detection unit detects the features in a portion of the captured image that corresponds to at least one of the face of the subject and a member disposed at the head of the subject.

11. An eyeglass lens selection system, comprising:

the image generating device according to any one of claims 1 through 10;
the display device; and
an image capturing unit that captures an image of the subject looking at the display device.

12. A method for image generation, comprising:

detecting features (F) in a captured image (Is) obtained by capturing an image of a subject looking at a display device (20) ;
calculating a distance between the subject and the display device and an orientation of a face of the subject based upon positions of the features having been detected and/or a distance between the features having been detected;
generating an image of a virtual visual field (21), which is a visual field as would be viewed by the subject looking at the display device through a virtual eyeglass lens (9), based upon virtual eyeglass lens data, the distance between the subject and the display device and the orientation of the face; and
causing the display device to display the image of the virtual visual field.

13. A program that enables a processing device to execute:

feature detection processing through which features (F) are detected in a captured image (Is) obtained by capturing an image of a subject (S) looking at a display device;
parameter calculation processing through which a distance between the subject and the display device and an orientation of a face of the subject are calculated based upon positions of the features having been detected and/or a distance between the features having been detected;
image generation processing through which an image of a virtual visual field (21) that is a visual field as would be viewed by the subject looking at the display device through a virtual eyeglass lens (9) is generated based upon virtual eyeglass lens data, the distance between the subject and the display device and the orientation of the face; and
display control processing through which the image of the virtual visual field is displayed at the display device.

**Patentansprüche**

1. Bilderzeugungsvorrichtung (1), umfassend:

    eine Merkmalserkennungseinheit (41), die Merkmale (F) in einem aufgenommenen Bild (Is) erkennt, das durch Aufnehmen eines Bildes eines Subjekts erhalten wird, das auf eine Anzeigevorrichtung (20) blickt;
    eine Parameterberechnungseinheit (42), die einen Abstand zwischen dem Subjekt und der Anzeigevorrichtung und eine Orientierung eines Gesichts des Subjekts auf der Grundlage von Positionen der Merkmale, die erkannt worden sind, und/oder eines Abstands zwischen den Merkmalen, die erkannt worden sind, berechnet;
    eine Bilderzeugungseinheit (43), die ein Bild eines virtuellen Sichtfelds, das ein Sichtfeld ist, wie es vom Subjekt beim Blicken auf die Anzeigevorrichtung durch ein virtuelles Brillenglas (9) betrachtet werden würde, auf der Grundlage von Daten des virtuellen Brillenglases, dem Abstand zwischen dem Subjekt und der Anzeigevorrichtung und der Orientierung des Gesichts erzeugt; und
    eine Anzeigesteuerungseinheit (44), die die Anzeigevorrichtung veranlasst, das Bild des virtuellen Sichtfelds anzuzeigen.

2. Bilderzeugungsvorrichtung nach Anspruch 1, wobei:

    die Parameterberechnungseinheit eine Augenposition in Bezug auf ein Auge des Subjekts auf der Grundlage der Positionen der Merkmale, die erkannt worden sind, und/oder des Abstands zwischen den Merkmalen, die erkannt worden sind, berechnet; und
    die Bilderzeugungseinheit ein Bild eines virtuellen Sichtfelds, das vom Subjekt beim Blicken auf die Anzeigevorrichtung durch das virtuelle Brillenglas von der Augenposition aus betrachtet werden soll, auf der Grundlage der Augenposition, des Abstands zwischen dem Subjekt und der Anzeigevorrichtung und der Orientierung des Gesichts erzeugt.

3. Bilderzeugungsvorrichtung nach Anspruch 2, wobei: die Bilderzeugungseinheit das Bild des virtuellen Sichtfelds, das vom Subjekt beim Blicken auf ein virtuelles Objekt betrachtet werden soll, das an einer von der Anzeigevorrichtung eingenommenen Position angeordnet ist, erzeugt.

4. Bilderzeugungsvorrichtung nach Anspruch 3, wobei: die Bilderzeugungseinheit einen dreidimensionalen virtuellen Raum konstruiert, der das virtuelle Objekt enthält, und einen Sichtwinkel des Bildes des virtuellen Sichtfelds entsprechend einer Größe eines Anzeigebildschirms der Anzeigevorrichtung unabhängig von der Augenposition anpasst.

5. Bilderzeugungsvorrichtung nach Anspruch 4, wobei: die Bilderzeugungseinheit auf der Grundlage einer Richtung, entlang welcher Licht, das das Auge verlassen hat, aus dem virtuellen Brillenglas austritt, eine dem virtuellen Brillenglas zuzuordnende lokale Scherung berechnet.

6. Bilderzeugungsvorrichtung nach Anspruch 4 oder Anspruch 5, wobei:
die Bilderzeugungseinheit eine Aberrationsgröße berechnet, welche das Ausmaß einer an einer Netzhaut des Auges auftretenden Aberration angibt, auf der Grundlage eines Ergebnisses, das durch Verfolgen von Lichtstrahlen erhalten worden ist, die von Punkten an dem in dem virtuellen Raum angeordneten virtuellen Objekt ausgegangen sind, die durch das virtuelle Brillenglas übertragen werden und in die Netzhaut eintreten und die das Bild des virtuellen Sichtfelds erzeugen, das durch eine auf der Grundlage der Aberrationsgröße ausgeführte Faltungsoperation unscharf gemacht ist.

7. Bilderzeugungsvorrichtung nach Anspruch 6, wobei: die Bilderzeugungseinheit die Aberrationsgröße auf der Grundlage von mindestens einem aus der Akkommodationsfähigkeit des Subjekts, dem Alter des Subjekts und den Verordnungsdaten betreffend das Subjekt berechnet.

8. Bilderzeugungsvorrichtung nach Anspruch 6 oder Anspruch 7, wobei:
die Bilderzeugungseinheit den Kern der Faltungsoperation auf der Grundlage des Abstands zwischen dem Subjekt und der Anzeigevorrichtung ändert.

9. Bilderzeugungsvorrichtung nach einem der Ansprüche 1 bis 8, wobei:
die Parameterberechnungseinheit auf der Grundlage von Verordnungsdaten betreffend das Subjekt und/oder Messwerten, die durch eine tatsächliche Messung erhalten worden sind, die an dem Subjekt durchgeführt worden ist, das das Brillenglas trägt, eine Position und eine Orientierung des virtuellen Brillenglases während des Erzeugens des Bildes des virtuellen Sichtfelds berechnet.

10. Bilderzeugungsvorrichtung nach einem der Ansprüche 1 bis 9, wobei:
die Merkmalserkennungseinheit die Merkmale in einem Abschnitt des aufgenommenen Bildes erkennt, der mindestens einem von dem Gesicht des Subjekts und einem am Kopf des Subjekts angeordneten Element entspricht.

**11.** Brillenglasauswahlsystem, umfassend:

die Bilderzeugungsvorrichtung nach einem der Ansprüche 1 bis 10;
die Anzeigevorrichtung; und
eine Bildaufnahmeeinheit, die ein Bild des Subjekts aufnimmt, das auf die Anzeigevorrichtung blickt.

**12.** Verfahren zur Bilderzeugung, umfassend:

Erkennen von Merkmalen (F) in einem aufgenommen Bild (Is), das durch Aufnehmen eines Bildes eines Subjekts erhalten wird, das auf eine Anzeigevorrichtung (20) blickt;
Berechnen eines Abstands zwischen dem Subjekt und der Anzeigevorrichtung und einer Orientierung eines Gesichts des Subjekts auf der Grundlage von Positionen der Merkmale, die erkannt worden sind, und/oder eines Abstands zwischen den Merkmalen, die erkannt worden sind;
Erzeugen eines Bildes eines virtuellen Sichtfelds (21), das ein Sichtfeld ist, wie es vom Subjekt beim Blicken auf die Anzeigevorrichtung durch ein virtuelles Brillenglas (9) betrachtet werden würde, auf der Grundlage von Daten des virtuellen Brillenglases, dem Abstand zwischen dem Subjekt und der Anzeigevorrichtung und der Orientierung des Gesichts; und
Veranlassen der Anzeigevorrichtung, das Bild des virtuellen Sichtfelds anzuzeigen.

**13.** Programm, das eine Verarbeitungsvorrichtung dazu befähigt, Folgendes auszuführen:

Merkmalserkennungsverarbeitung, durch die Merkmale (F) in einem aufgenommen Bild (Is) erkannt werden, das durch Aufnehmen eines Bildes eines Subjekts (S) erhalten wird, das auf eine Anzeigevorrichtung blickt;
Parameterberechnungsverarbeitung, durch die ein Abstand zwischen dem Subjekt und der Anzeigevorrichtung und eine Orientierung eines Gesichts des Subjekts auf der Grundlage von Positionen der Merkmale, die erkannt worden sind, und/oder eines Abstands zwischen den Merkmalen, die erkannt worden sind, berechnet werden;
Bilderzeugungsverarbeitung, durch die auf der Grundlage von Daten des virtuellen Brillenglases, dem Abstand zwischen dem Subjekt und der Anzeigevorrichtung und der Orientierung des Gesichts ein Bild eines virtuellen Sichtfelds (21), das ein Sichtfeld ist, wie es vom Subjekt beim Blicken auf die Anzeigevorrichtung durch ein virtuelles Brillenglas (9) betrachtet werden würde, erzeugt wird; und

Anzeigesteuerungsverarbeitung, durch die das Bild des virtuellen Sichtfelds an der Anzeigevorrichtung angezeigt wird.

**Revendications**

**1.** Dispositif de génération d'image (1), comprenant :

une unité de détection de caractéristiques (41) qui détecte des caractéristiques (F) dans une image capturée (Is) obtenue en capturant une image d'un sujet regardant un dispositif d'affichage (20) ;
une unité de calcul de paramètres (42) qui calcule une distance entre le sujet et le dispositif d'affichage et une orientation d'un visage du sujet sur la base de positions des caractéristiques ayant été détectées et/ou d'une distance entre les caractéristiques ayant été détectées ;
une unité de génération d'image (43) qui génère une image d'un champ visuel virtuel, qui est un champ visuel tel qu'il serait vu par le sujet regardant le dispositif d'affichage à travers un verre de lunettes virtuel (9), sur la base de données relatives au verre de lunettes virtuel, de la distance entre le sujet et le dispositif d'affichage et de l'orientation du visage ; et
une unité de commande d'affichage (44) qui amène le dispositif d'affichage à afficher l'image du champ visuel virtuel.

**2.** Dispositif de génération d'image selon la revendication 1, dans lequel :

l'unité de calcul de paramètres calcule une position de l'œil par rapport à un œil du sujet sur la base des positions des caractéristiques ayant été détectées et/ou de la distance entre les caractéristiques ayant été détectées ; et,
l'unité de génération d'image génère une image d'un champ visuel virtuel devant être vu par le sujet regardant le dispositif d'affichage à travers le verre de lunettes virtuel à partir de la position de l'œil, sur la base de la position de l'œil, de la distance entre le sujet et le dispositif d'affichage et de l'orientation du visage.

**3.** Dispositif de génération d'image selon la revendication 2, dans lequel :
l'unité de génération d'image génère l'image du champ visuel virtuel devant être vue par le sujet regardant un objet virtuel situé à une position prise par le dispositif d'affichage.

**4.** Dispositif de génération d'image selon la revendication 3, dans lequel :
l'unité de génération d'image construit un espace virtuel tridimensionnel qui contient l'objet et ajuste un angle de vue de l'image du champ visuel virtuel en correspondance avec une taille d'un écran d'affichage du dispositif d'affichage indépendamment de la position de l'œil.

5. Dispositif de génération d'image selon la revendication 4, dans lequel :
l'unité de génération d'image calcule une obliquité locale attribuable au verre de lunettes virtuel sur la base d'une direction le long de laquelle une lumière ayant quitté l'œil sort du verre de lunettes.

6. Dispositif de génération d'image selon la revendication 4 ou la revendication 5, dans lequel :
l'unité de génération d'image calcule une quantité d'aberration indiquant une étendue d'aberration se produisant au niveau d'une rétine de l'œil sur la base d'un résultat obtenu en traçant des rayons lumineux, ayant quitté des points sur l'objet placé dans l'espace virtuel,
transmis à travers le verre de lunettes et entrant dans la rétine, et génère l'image du champ visuel virtuel qui est floutée au moyen d'une opération de convolution exécutée sur la base de la quantité d'aberration.

7. Dispositif de génération d'image selon la revendication 6, dans lequel :
l'unité de génération d'image calcule la quantité d'aberration sur la base d'au moins un parmi la capacité d'accommodation du sujet, un âge du sujet et des données de prescription relatives au sujet.

8. Dispositif de génération d'image selon la revendication 6 ou la revendication 7, dans lequel :
l'unité de génération d'image modifie un noyau de l'opération de convolution sur la base de la distance entre le sujet et le dispositif d'affichage.

9. Dispositif de génération d'image selon l'une quelconque des revendications 1 à 8, dans lequel :
l'unité de calcul de paramètres calcule une position et une attitude du verre de lunettes virtuel tout en générant l'image du champ visuel virtuel sur la base de données de prescription relatives au sujet et/ou de valeurs de mesure obtenues par une mesure réelle effectuée sur le sujet portant le verre de lunettes.

10. Dispositif de génération d'image selon l'une quelconque des revendications 1 à 9, dans lequel :
l'unité de détection de caractéristiques détecte les caractéristiques dans une portion de l'image capturée qui correspond à au moins un parmi le visage du sujet et un organe disposé au niveau de la tête du sujet.

11. Système de sélection de verre de lunettes, comprenant :

le dispositif de génération d'image selon l'une quelconque des revendications 1 à 10 ;
le dispositif d'affichage ; et,
une unité de capture d'images qui capture une image du sujet regardant le dispositif d'affichage.

12. Procédé de génération d'image, comprenant les étapes consistant à :

détecter des caractéristiques (F) dans une image capturée (Is) obtenue en capturant une image d'un sujet regardant un dispositif d'affichage (20) ;
calculer une distance entre le sujet et le dispositif d'affichage et une orientation d'un visage du sujet sur la base de positions des caractéristiques ayant été détectées et/ou d'une distance entre les caractéristiques ayant été détectées ;
générer une image d'un champ visuel virtuel (21), qui est un champ visuel tel qu'il serait vu par le sujet regardant le dispositif d'affichage à travers un verre de lunettes virtuel (9), sur la base de données relatives au verre de lunettes virtuel, de la distance entre le sujet et le dispositif d'affichage et de l'orientation du visage ; et
amener le dispositif d'affichage à afficher l'image du champ visuel virtuel.

13. Programme qui permet à un dispositif de traitement d'exécuter les étapes suivantes :

traitement de détection de caractéristiques au moyen duquel des caractéristiques (F) sont détectées dans une image capturée (Is) obtenue en capturant une image d'un sujet (S) regardant un dispositif d'affichage ;
traitement de calcul de paramètres au moyen duquel une distance entre le sujet et le dispositif d'affichage et une orientation d'un visage du sujet sont calculées sur la base de positions des caractéristiques ayant été détectées et/ou d'une distance entre les caractéristiques ayant été détectées ;
traitement de génération d'image au moyen duquel une image d'un champ visuel virtuel (21) qui est un champ visuel tel qu'il serait vu par le sujet regardant le dispositif d'affichage à travers un verre de lunettes virtuel (9) est générée sur la base de données relatives au verre de lunettes virtuel, de la distance entre le sujet et le dispositif d'affichage et de l'orientation du visage ; et
traitement de commande d'affichage au moyen de laquelle l'image du champ visuel virtuel est affichée au niveau du dispositif d'affichage.

FIG. 1

# FIG. 2

(A)

(B)

# FIG. 3

(A)

(B)

# FIG. 4

(A)

(B)

# FIG. 5

(A)

(B)

# FIG.6

(A)

(B)

# FIG. 7

# FIG. 8

# FIG. 9

(A)

PRE-BLUR IMAGE                    PSF                  BLURRED IMAGE

(B)

PRE-BLUR IMAGE                    PSF                  BLURRED IMAGE

# FIG. 10

|  | K1 | | | K2 | | | K3 | | | K4 | | | K5 | | | K6 | | |
|---|---|---|---|---|---|---|---|---|---|---|---|---|---|---|---|---|---|---|

K

| 0.02 | 0.12 | 0.06 |
|---|---|---|
| 0.12 | 0.36 | 0.12 |
| 0.06 | 0.12 | 0.02 |

PSF1

| 0.00 | 0.08 | 0.04 |
|---|---|---|
| 0.08 | 0.60 | 0.08 |
| 0.04 | 0.08 | 0.00 |

PSF2

| 0.00 | 0.04 | 0.00 |
|---|---|---|
| 0.04 | 0.84 | 0.04 |
| 0.00 | 0.04 | 0.00 |

PSF3

| 0.00 | 0.00 | 0.00 |
|---|---|---|
| 0.00 | 1.00 | 0.00 |
| 0.00 | 0.00 | 0.00 |

PSF4

| 0.00 | 0.04 | 0.00 |
|---|---|---|
| 0.04 | 0.84 | 0.04 |
| 0.00 | 0.04 | 0.00 |

PSF5

| 0.04 | 0.08 | 0.00 |
|---|---|---|
| 0.08 | 0.60 | 0.08 |
| 0.00 | 0.08 | 0.04 |

PSF6

ACCOMMODATION

# FIG. 11

START

S1001

CORRECT VISUAL ACUITY OF SUBJECT

S1003

SET VIRTUAL OBJECTS IN VIRTUAL SPACE

S1005

OBTAIN EYEGLASS LENS DESCRIPTION DATA AND DETERMINE POSITION OF VIRTUAL EYEGLASS LENS RELATIVE TO SUBJECT WEARING VIRTUAL EYEGLASS LENS

S1007

CAPTURE IMAGE OF SUBJECT LOOKING AT DISPLAY UNIT AND EXTRACT FEATURES FROM SUBJECT IMAGE OBTAINED THROUGH IMAGE CAPTURING OPERATION

S1009

CALCULATE DISTANCE BETWEEN SUBJECT AND DISPLAY UNIT AND ORIENTATION OF SUBJECT'S FACE BASED UPON POSITIONS OF EXTRACTED FEATURES AND DISTANCES BETWEEN EXTRACTED FEATURES

S1011

GENERATE IMAGE OF VIRTUAL VISUAL FIELD (VISUAL FIELD IMAGE) TO BE VIEWED BY SUBJECT LOOKING AT DISPLAY UNIT THROUGH EYEGLASS LENS, BASED UPON DISTANCE BETWEEN SUBJECT AND DISPLAY UNIT AND FACE ORIENTATION

S1013       YES

INSTRUCTION FOR SWITCHING EYEGLASS LENS ENTERED?

NO

S1015

NO

INSTRUCTION FOR ENDING VISUAL FIELD IMAGE GENERATION ENTERED?

YES

S1017

OBTAIN RESPONSE FROM SUBJECT HAVING VIEWED VISUAL FIELD IMAGE AND SELECT EYEGLASS LENS TO BE WORN BY SUBJECT BASED UPON RESPONSE

S1019

PLACE ORDER FOR EYEGLASS LENS TO BE WORN BY SUBJECT AND ENGAGE EYEGLASS LENS PROCESSING MACHINE IN MANUFACTURE

END

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6023801 B
- WO 2013067230 A
- US 2016270656 A1
- US 2017052393 A

**Non-patent literature cited in the description**

- Computing 3-D Head Orientation from a Monocular Image Sequence. **T. HORPRASERT** ; **Y. YACOOB** ; **L. DAVIS**. Proceedings of the Second International Conference on Automatic Face and Gesture Recognition (US). IEEE, 1996, 242-247